**Europäisches Patentamt**

**European Patent Office**

**Office européen des brevets**

(11) Veröffentlichungsnummer: **0 149 456**
**B1**

(12) **EUROPÄISCHE PATENTSCHRIFT**

(45) Veröffentlichungstag der Patentschrift:
08.02.89

(51) Int. Cl.⁴: **C 07 C 69/67, C 07 C 67/30**

(21) Anmeldenummer: **85100067.9**

(22) Anmeldetag: **03.01.85**

(54) Verfahren zur Herstellung eines Glyoxylsäureesters.

(30) Priorität: **14.01.84 DE 3401115**

(43) Veröffentlichungstag der Anmeldung:
**24.07.85 Patentblatt 85/30**

(45) Bekanntmachung des Hinweises auf die Patenterteilung:
**08.02.89 Patentblatt 89/6**

(84) Benannte Vertragsstaaten:
**AT BE CH DE FR GB IT LI NL**

(56) Entgegenhaltungen:
**EP-A- 0 008 013**
**DE-A- 2 902 779**

(73) Patentinhaber: **HOECHST AKTIENGESELLSCHAFT,**
**Postfach 80 03 20, D-6230 Frankfurt am Main 80 (DE)**

(72) Erfinder: **Driscoll, Robert Kenneth, Dr.,**
**Heilmannstrasse 43, D-6000 Frankfurt am Main 50 (DE)**
Erfinder: **Leupold, Ernst Ingo, Dr., Am Zäunefeld 15,**
**D-6392 Neu-Anspach (DE)**
Erfinder: **Ebertz, Wolfgang, Dr., Bismarckstrasse 25,**
**D-5300 Bonn 1 (DE)**
Erfinder: **Wunder, Friedrich, Dr., Jahnstrasse 46,**
**D-6093 Flörsheim am Main (DE)**

**Beschreibung**

Aus DE-PS 2 904 775 und DE-OS 3 323 372 ist bekannt, dass sich Glykolsäureester an heterogenen Katalysatoren in der Gasphase zu Glyoxylsäureestern oxydehydrieren lassen. Diese Reaktion ist exotherm, ausserdem wird viel Wärme durch die als Neben- bzw. Folgereaktionen auftretende Totaloxidation zu $CO_2$ und Wasser freigesetzt. Das üblicherweise verwendete, mit körnigem Trägerkatalysator gefüllte Reaktorrohr hat daher im allgemeinen einen reaktiv geringen Durchmesser, um eine gute Wärmeabfuhr zu ermöglichen. Um unerwünschte Folgereaktionen zu unterdrücken, arbeitet man im allgemeinen mit kurzen Verweilzeiten von 0,05 bis 1,0 Sekunden. Solche Verweilzeiten werden mit hohem Gasdurchsatz erreicht. Es müssen ausserdem grosse Mengen inertes Trägergas, z.B. Stickstoff, dem Reaktor zugeführt werden, um den Sauerstoffanteil in der Gasphase unter der Explosionsgrenze zu halten und die freigesetzte Reaktionswärme vom Katalysator auf die gekühlte Reaktorwand zu übertragen. Diese Kombination von engem Rohr und hohem Gasdurchsatz führt zu beträchtlichen Druckverlusten in einem aus körnigem Material bestehenden Katalysatorbett. In der Praxis wird das Verdünnungsgas (ca. 95% der Gasphase) im Kreis gefahren. Der grosse Druckverlust verursacht einen hohen Verbrauch an Verdichtungsarbeit, um das Verdünnungsgas wieder verwenden zu können. Ziel der vorliegenden Erfindung ist es, diesen Nachteil zu überwinden.

Die vorliegende Erfindung betrifft demgemäss ein Verfahren zur Herstellung eines Glyoxylsäureesters durch Oxydehydrierung des entsprechenden Glykolsäureesters in der Gasphase an einem Trägerkatalysator in einem rohrförmigen Reaktor, dadurch gekennzeichnet, dass der Katalysatorträger aus mindestens einem zylinderförmigen Monolith besteht, wobei der Monolith im wesentlichen den gleichen Durchmesser wie das Reaktorrohr hat und Kanäle mit einem Durchmesser von 1 bis 10 mm enthält, die vom Eingang zum Ausgang des Reaktorrohrs führen und wobei 60 bis 90% des Monolithvolumens von Hohlräumen gebildet wird.

Hierbei wird ein «Monolith» definiert als ein grossstückiger, Kanäle enthaltender Träger aus keramischem Material. Die Kanäle können parallel oder geneigt zur Achse des Reaktorrohrs verlaufen. Vorzugsweise haben sie einen Durchmesser von 1–5 mm.

Bevorzugt werden mehrere zylinderförmige Monolithe im Reaktorrohr hintereinander angeordnet oder aufeinandergestapelt, da dies einfacher ist als einen einzigen Monolith mit derselben Länge wie das Reaktorrohr herzustellen und einzubauen. Diese Zylinder können sich wiederum aus einzelnen Segmenten zusammensetzen.

Monolithe mit Kanälen, die parallel zur Achse des Reaktorrohres verlaufen, werden bisher schon bei der Entgiftung von Autoabgasen verwendet (Ullmann, Encyklopädie der technischen Chemie, Band 13, S. 561). Solche Monolithe können auch beim vorliegenden Verfahren eingesetzt werden. Die Kanäle können dabei einen runden oder eckigen Querschnitt haben; bei eckigem Querschnitt spricht man auch von «Wabenkörpern». Ein solcher Monolith mit Kanälen parallel zur Reaktorachse führt wenig Reaktionswärme ab. Um Überhitzung zu vermeiden, wird man daher das Reaktionsrohr vorzugsweise in mehrere mit Monolith gefüllte Reaktionszonen unterteilen und Kühlzonen zwischen den Reaktionszonen einbauen (Hordenreaktor).

Bevorzugt verwendet man beim erfindungsgemässen Verfahren Monolithe mit Kanälen, die in der Mehrzahl nicht parallel zur Achse des Reaktorrohres verlaufen, sondern einen Winkel von 20 bis 70° mit der Achse bilden. Dabei müssen die Kanäle des Monoliths oder der Monolithe so zueinander passen, dass das eingesetzte Gas vom Eingang des Reaktorrohres zum Ausgang fliessen kann. Denn wegen der Neigung der Kanäle gegen die Reaktorachse treffen die Gase auf die Reaktorwand, und es muss natürlich sichergestellt sein, dass sie von dort durch einen anderen Kanal weiterfliessen können. Dasselbe gilt für den Übergang von einem Monolith zum anderen oder von einem Monolithsegment zum anderen.

Mit anderen Worten: die Kanalstruktur des Gesamtkatalysators muss in jedem Fall so sein, dass die Gase mit möglichst geringem Widerstand vom Eingang zum Ausgang des Reaktorrohrs gelangen können. Da die Gase bei ihrem Weg durch den Katalysator auf die kühlere Reaktorwand treffen, ist die Wärmeabfuhr einfacher als im Fall der Monolithe mit Kanälen parallel zur Reaktorachse. Ebenso wie dort können die Kanäle hier einen runden oder eckigen Querschnitt haben.

Besonders bevorzugt sind unter den genannten Monolithen mit geneigten Kanälen solche, die eine geordnete, sich stetig wiederholende Hohlstruktur in allen drei Raumrichtungen aufweisen. Solche Strukturen sind bereits bekannt bei sogenannten «statischen Mischern» (Chem. Ing. Tech. 51 (1979), S. 347–364).

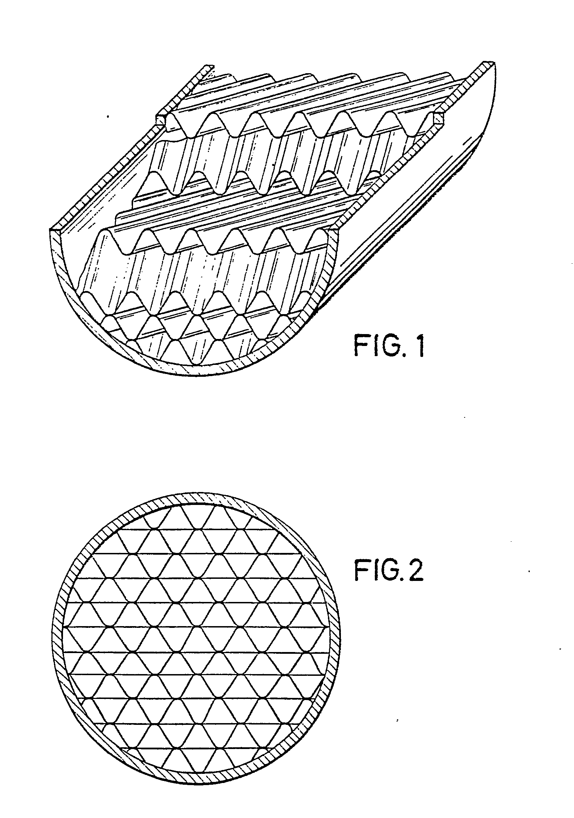

Insbesondere bevorzugt sind Monolithe mit einer durch Figur 1 und 2 illustrierten Struktur, die ebenfalls schon von statischen Mischern her bekannt ist. Solche Monolithe, die im folgenden auch als Monolithe Typ I bezeichnet werden, bestehen aus parallel zur Reaktorachse angeordneten, aufeinander liegenden gewellten Schichten (also etwa wie Wellblech geformt), wobei die Wellentäler in aufeinander folgenden Schichten alternierend einen Winkel von $\alpha$ und $-\alpha$ mit der Reaktorachse bilden ($\alpha = 20$–$70°$). Die durch die Wellentäler und Wellenkämme der aufeinanderliegenden Schichten gebildeten Kanäle haben den Querschnitt eines Dreiecks mit stark abgerundeten Ekken. Zwischen den aufeinander liegenden gewellten Schichten existiert an jeder Berührungsstelle eine feste Verbindung, so dass jedes Monolithelement eine einzige mit Kanälen durchzogene Hohlraumstruktur darstellt. Solche Monolithe sind im Handel unter dem Namen «Sulzer-Packung» erhältlich. Wie für den allgemeineren Fall oben erwähnt, werden auch hier die Monolithe bevorzugt in Form von mehreren aufeinander gestapelten Zylindern eingesetzt. Die beschriebene Struktur stellt dabei automatisch sicher, dass die Kanäle der aufeinander gestapelten Zylinder so zueinander passen, dass das eingesetzte Gas vom Eingang des Reaktorrohrs zum Ausgang fliessen kann.

Überraschenderweise bringt der erfindungsgemässe Einsatz eines Monolith-Trägers eine deutlich bessere Ausbeute und Raum-Zeit-Ausbeute als der Einsatz einer konventionellen Schüttung aus kugelförmigen Katalysatorteilchen. Ausserdem erzeugt die erfindungsgemässe Monolithfüllung einen wesentlich kleineren Druckverlust als eine Kugelschüttung.

Der erfindungsgemässe Einsatz von Monolithen hat gegenüber dem Einsatz eines Kugelbetts folgende weitere Vorteile: Der Einbau und der Wechsel des Katalysators wird beträchtlich erleichtert, da das Katalysatorbett nur aus wenigen Teilen besteht. Ausserdem wird der Abrieb der Aktivkomponenten wesentlich reduziert.

Als keramisches Material für die Monolithe eignen sich Silikate, Aluminiumoxide, Aluminiumsilikate oder Siliciumcarbid, vorzugsweise mit BET-Oberflächen von weniger als 50 $m^2g^{-1}$. Besonders bevorzugt werden Monolithe, die überwiegend aus Aluminiumsilikat bestehen.

Als katalytisch aktive Komponenten kommen im Prinzip alle für Oxydehydrierungsreaktionen geeigneten Elemente in Frage. Der Katalysator enthält jedoch vorzugsweise mindestens eines der Elemente V, Au, Mo, Ag, Cu, Sn, Sb, Bi und P. Jedoch zeigen auch andere Elemente der 3. bis 5. Hauptgruppe eine katalytische Wirkung.

Die genannten Elemente werden entweder in metallischer Form oder in Form ihrer Verbindungen, z.B. als Oxide, Nitrate, Acetate, Acetylacetonate, Oxalate, Citrate oder Halogenide in die Reaktionszone eingebracht.

Die Gesamtmenge an Elementen auf dem Träger kann in weiten Grenzen schwanken. Im allgemeinen beträgt sie 0,01 bis 50 Gew.-%, vorzugsweise 0,1 bis 20 Gew.-%, bezogen auf die Gesamtmasse des Trägerkatalysators. Die katalytisch aktiven Komponenten werden zweckmässig in Form einer Lösung auf den Träger gebracht, dann wird das Lösemittel abgedampft und der Katalysator getrocknet. Als Lösemittel verwendet man im allgemeinen Wasser, Salzsäure, Salpetersäure, Alkalilaugen oder wässrige Ammoniaklösung, vorzugsweise Wasser oder Salzsäure.

Es hat sich als vorteilhaft erwiesen, vor dem Einleiten des Glykolsäureesters in die Reaktionszone ein oxydierendes Gas, insbesondere Sauerstoff oder Luft, oder ein reduzierendes Gas, insbesondere Wasserstoff oder mit Inertgas verdünnten Wasserstoff, bei Temperaturen von 100 bis 800 °C, insbesondere 300 bis 600 °C, über den Katalysator zu leiten.

Bei dem erfindungsgemässen Verfahren werden Glykolsäureester der allgemeinen Formel HO–CH$_2$–COOR in Dampfform eingesetzt.

Dabei ist R ein Kohlenwasserstoffrest, vorzugsweise ein aliphatischer, geradkettiger oder verzweigter Alkylrest mit 1 bis 8 C-Atomen, insbesondere mit 1 bis 5 C-Atomen.

Die gasförmigen Glykolsäureester werden zusammen mit Sauerstoff oder einem sauerstoffhaltigen Gas, wie Luft, über den Katalysator geleitet. Vorzugsweise verdünnt man mit einem Trägergas, wie Stickstoff oder Edelgasen.

Bei dem erfindungsgemässen Verfahren setzt man pro Mol Glykolsäureester im allgemeinen folgende Mengen an Zusatzstoffen ein:

Sauerstoff: 0,1 bis 5 Mol, vorzugsweise 0,5 bis 3 Mol.

Trägergas: 0 bis 200 Mol, vorzugsweise 30 bis 100 Mol.

Auch ausserhalb dieser Grenzen werden noch zufriedenstellende Ergebnisse erzielt.

Die Oxydehydrierung wird im allgemeinen bei Temperaturen zwischen 100 und 600 °C, vorzugsweise zwischen 200 und 400 °C durchgeführt. Die Verweilzeit liegt vorzugsweise zwischen 0,05 und 10 Sekunden, insbesondere jedoch zwischen 0,05 und 1 Sekunde. Auch ausserhalb dieser Grenzen erhält man noch befriedigende Ergebnisse.

Die Oxydehydrierung wird bevorzugt bei Normaldruck durchgeführt, jedoch können auch verminderte oder erhöhte Drucke angewendet werden, d.h. 0,01 bis 100 bar.

Im einzelnen geht man so vor, dass der Glykolsäureester und Sauerstoff oder sauerstoffhaltiges Gas, sowie ggf. das Trägergas, aus Dosierungsvorrichtungen in eine Verdampfungszone und die entstandene Gasmischung dann durch ein von aussen beheiztes und mit dem Katalysator gefülltes Reaktionsrohr geleitet wird. Es hat sich dabei als vorteilhaft erwiesen, den Sauerstoff oder das sauerstoffhaltige Gas sowie das Trägergas vor der Einleitung in den Reaktor auf die Reaktionstemperatur aufzuheizen.

Die nachfolgenden Beispiele sollen die Erfindung näher erläutern.

Beispiel 1

Der Versuch wurde in einem senkrecht angeordneten V4A-Stahlrohrreaktor von 1500 mm Länge und 27 mm Durchmesser durchgeführt. Der Reaktor wurde von aussen beheizt, und die Temperatur im Inneren wird mit Hilfe mehrerer Thermoelemente gemessen.

Der Katalysatorträger bestand aus 9 aufeinandergeschichteten zylinderförmigen Monolithstücken des Typs I. Jedes Stück hatte einen Durchmesser von 26 mm, eine Länge von 50 mm und ca. 3 mm Kanaldurchmesser. Zentral durch die Monolith-Körper war eine Bohrung von 9 mm Durchmesser durchgeführt, in welche Thermoelemente zur Temperaturmessung eingepasst waren. Die Monolithe bestanden aus mit Aluminiumoxid beschichtetem Aluminiumsilikat mit ca. 20 $m^2g^{-1}$ BET-Oberfläche. Der Katalysatorträger füllte ein Volumen von 210 ml im Reaktorrohr aus. Dieses Volumen wird im folgenden als Katalysatorvolumen bezeichnet.

Zur Herstellung des Katalysators löste man 16,4 g Vanadiumpentoxid in 150 ml konzentrierter Salzsäure. Jedes Monolithstück wurde durch Eintauchen in diese Lösung getränkt. Das Lösungsmittel wurde auf einem Dampfbad abgedampft und der Monolith anschliessend bei 110 °C getrocknet. Dieses Verfahren wurde wiederholt, bis die gewünschte Vanadium-Konzentration, nämlich 25,8 g Vanadium pro Liter Katalysatorvolumen erreicht war.

In gleicher Weise wurden 54,3 g Silber pro Liter Katalysatorvolumen auf den bereits mit Vanadium getränkten Monolith durch Eintauchen in eine 1,8 molare wässrige Lösung von Silbernitrat aufgebracht.

Die Aktivierung des gesamten Katalysators wurde in einem Gasstrom aus 2,4 mol/h Sauerstoff und 91,6 mol/h Stickstoff drei Stunden lang bei 400 °C durchgeführt.

184,8 ml/h Glykolsäuremethylester (2,4 mol/h), 2,4 mol/h Sauerstoff und 91,6 mol/h Stickstoff wurden gleichzeitig in einen Verdampfungstopf eingeleitet. Die gesamte Gasphase wurde vor Einleitung in den senkrecht angeordneten Reaktor auf 275 °C aufgeheizt. Die Reaktionstemperatur im Reaktorrohr betrug ebenfalls 275 °C. Nach einer Anlaufzeit von 1 Stunde zur Einstellung konstanter Betriebsbedingungen wurde der Versuch über einen Zeitraum von 4 Stunden fortgeführt.

Die folgenden Ergebnisse wurden erhalten:

| | | |
|---|---|---|
| Glyoxylsäuremethylester-Ausbeute | = | 82,1% |
| Raum-Zeit-Ausbeute | = | 825,3 g/l·h |
| Glykolsäuremethylester-Umsatz | = | 94,6% |
| Glykolsäuremethylester-Selektivität | = | 86,8% |
| Druckverlust im Katalysatorbett | | < 1 mbar |

Vergleichsbeispiel

Der Katalysatorträger bestand aus Aluminiumsilikatkugeln (1 mm Durchmesser) mit 1 m²g⁻¹ BET-Oberfläche. Der Katalysator enthielt 38,5 g Vanadium und 81,1 g Silber pro Liter Katalysatorvolumen. Zur Herstellung des Katalysators löste man 14,4 g Vanadium-Pentoxid in 109 ml konzentrierter Salzsäure, tränkte 210 ml Katalysator mit dieser Lösung und dampfte das Lösungsmittel auf einem Dampfbad ab. In gleicher Weise wurden 26,8 g Silbernitrat nach Lösen in 43,7 ml Wasser aufgebracht. Der Katalysator wurde anschliessend bei 110 °C getrocknet. Diese 210 ml Katalysator wurden in denselben senkrecht angeordneten Stahlreaktor eingefüllt und in gleicher Weise aktiviert wie in Beispiel 1.

Genau so wie in Beispiel 1 wurden die dort angegebenen Mengen an Reaktionsgasen durch diese Katalysatorschüttung bei derselben Reaktionstemperatur geleitet.

Es wurden die folgenden Ergebnisse erhalten:

| | | |
|---|---|---|
| Glyoxylsäuremethylester-Ausbeute | = | 75% |
| Raum-Zeit-Ausbeute | = | 754,3 g/l·h |
| Glykolsäuremethylester-Umsatz | = | 87,8% |
| Glykolsäuremethylester-Selektivität | = | 85,4% |

| | | |
|---|---|---|
| Druckverlust im Katalysatorbett | = | 360 mbar |

Der Druckverlust ist wesentlich höher als im Beispiel 1 und die Katalysatorleistung etwas verringert.

## Patentansprüche

1. Verfahren zur Herstellung eines Glyoxylsäureesters durch Oxydehydrierung des entsprechenden Glykolsäureesters in der Gasphase an einem Trägerkatalysator in einem rohrförmigen Reaktor, dadurch gekennzeichnet, dass der Katalysatorträger aus mindestens einem zylinderförmigen Monolith besteht, wobei der Monolith im wesentlichen den gleichen Durchmesser wie das Reaktorrohr hat und Kanäle mit einem Durchmesser von 1 bis 10 mm enthält, die vom Eingang zum Ausgang des Reaktorrohrs führen und wobei 60 bis 90% des Monolithvolumens von Hohlräumen gebildet wird.

2. Verfahren nach Anspruch 1, dadurch gekennzeichnet, dass die Kanäle einen Durchmesser von 1–5 mm haben.

3. Verfahren nach Anspruch 1 oder 2, dadurch gekennzeichnet, dass mehrere zylinderförmige Monolithe aufeinandergestapelt oder hintereinander angeordnet werden.

4. Verfahren nach einem der Ansprüche 1–3, dadurch gekennzeichnet, dass die Kanäle in der Mehrzahl einen Winkel von 20 bis 70° mit der Reaktorachse bilden.

5. Verfahren nach Anspruch 4, dadurch gekennzeichnet, dass die Monolithe eine geordnete, sich stetig wiederholende Hohlstruktur in allen drei Raumrichtungen haben.

6. Verfahren nach Anspruch 5, dadurch gekennzeichnet, dass die Monolithe aus parallel zur Reaktorachse angeordneten, aufeinander liegenden gewellten Schichten bestehen, wobei die Wellentäler in aufeinander folgenden Schichten alternierend einen Winkel von α und −α mit der Reaktorachse bilden und α einen Wert zwischen 20 und 70° hat.

## Claims

1. A process for the preparation of a glyoxylic acid ester by oxydehydrogenation of the corresponding glycolic acid ester in the gas phase over a supported catalyst in a tubular reactor, wherein the catalyst support consists of at least one cylindrical monolith, the monolith essentially having the same diameter as the reactor tube and containing channels 1 to 10 mm in diameter which lead from the inlet to the outlet of the reactor tube, and wherein 60 to 90% of the volume of the monolith is formed by hollow spaces.

2. The process as claimed in claim 1, wherein the channels have a diameter of 1–5 mm.

3. The process as claimed in either one of claims 1 and 2, wherein several cylindrical monoliths are stacked one on top of the other or arranged one behind the other.

4. The process as claimed in any one of claims 1 to 3, wherein most of the channels form an angle of 20 to 70° with the axis of the reactor.

5. The process as claimed in claim 4, wherein the monoliths have an ordered, continuously recurring hollow structure in all three spatial directions.

6. The process as claimed in claim 5, wherein the monoliths consist of corrugated layers arranged parallel to the axis of the reactor and placed one on top of the other, the troughs in subsequent layers alternately forming an angle of α and −α with the axis of the reactor and α having a value between 20 and 70°.

**Revendications**

1. Un procédé de préparation d'esters de l'acide glyoxylique par oxydéshydrogénation de l'ester glycolique correspondant en phase gazeuse sur un catalyseur sur support, dans un réacteur tubulaire, procédé caractérisé en ce que le support du catalyseur est formé d'un ou de plusieurs monolithes cylindriques ayant pratiquement le même diamètre que le réacteur tubulaire et comportant des canaux de 1 à 10 mm de diamètre qui vont de l'entrée à la sortie du réacteur, de 60 à 90% du volume du monolithe constituant des espaces vides.

2. Procédé selon la revendication 1, caractérisé en ce que les canaux ont un diamètre de 1 à 5 mm.

3. Procédé selon la revendication 1 ou 2 caractérisé en ce que plusieurs monolithes cylindriques sont empilés les uns sur les autres ou disposés l'un derrière l'autre.

4. Procédé selon l'une quelconque des revendications 1 à 3, caractérisé en ce que la majorité des canaux font un angle de 20 à 70° avec l'axe du réacteur.

5. Procédé selon la revendication 4 caractérisé en ce que les monolithes ont dans les trois directions de l'espace une structure creuse ordonnée qui se répète constamment.

6. Procédé selon la revendication 5 caractérisé en ce que les monolithes sont formés de couches plissées superposées parallèles à l'axe du réacteur, et les creux des plis des couches successives forment en alternance un angle α et −α avec l'axe du réacteur, l'angle α étant un angle de 20 à 70°.

FIG. 1

FIG.2